(19) Europäisches Patentamt
European Patent Office
Office européen des brevets

(11) Publication number : 0 452 257 A2

# (12) EUROPEAN PATENT APPLICATION

(21) Application number : 91810219.5

(22) Date of filing : 26.03.91

(51) Int. Cl.[5] : C07K 5/06, C07K 5/08, C07K 5/10, C07K 5/02, A61K 37/02, A61K 37/64

(30) Priority : 05.04.90 GB 9007751

(43) Date of publication of application : 16.10.91 Bulletin 91/42

(84) Designated Contracting States : AT BE CH DE DK ES FR GB GR IT LI LU NL SE

(71) Applicant : CIBA-GEIGY AG
Klybeckstrasse 141
CH-4002 Basel (CH)

(72) Inventor : Allen, Mark Christopher
32 Guildford Road
Horsham, West Sussex RH12 1LS (GB)
Inventor : Kane, Peter Daniel
9 Chequers Court, Ayshe Court Drive
Horsham, West Sussex (GB)
Inventor : Wade, Roy
5 Fordingbridge Close
Horsham, West Sussex RH12 1JN (GB)

(74) Representative : Sharman, Thomas et al
c/o CIBA-GEIGY PLC Patents Department
Tenax Road Trafford Park
Manchester M17 1WT (GB)

## (54) Novel platelet-aggregation inhibitors.

(57) A compound having the formula I :

$$X—(Y)_x—Asp—Z \qquad (I)$$

in which X is a group of formula :

$$R_1—(CH_2)_m—R_2—(CH_2)_n—CH(R_3)—CO—$$

in which $R_1$ is $NHR_4$ in which $R_4$ is H, acyl, alkoxycarbonyl or acyl, $R_2$ is arylene, heteroarylene, alkenylene, $CH_2$, O or NH, or any conbination of these, $R_3$ is H, $NH_2$ or NH-acyl, in the R- or S-configuration, and m and n are each O or an integer from 1 to 5 ; Y is a group of formula :

$$NH—(CH_2)_y—CO$$

in which y is an integer ranging from 1 to 12 ; Z is $NH_2$ or the radical of any of the common alpha-amino acids, or of the amides of such amino acids ; and pharmacologically - compatible salts of the compound of formula I ; provided that, when Y is Gly, X is other than Lys. The compounds of formula I are useful as platelet aggregation inhibitor.

EP 0 452 257 A2

The present invention relates to novel platelet - aggregation inhibitors.

Platelets are cellular elements which are found in blood and which participate in blood coagulation processes. Fibrinogen, a glycoprotein present in blood plasma, participates in platelet aggregation and in fibrin formation in the blood clotting mechanism. When a blood vessel receives an injury, the platelets binding to fibrinogen initiate aggregation and form a thrombus. Compounds which inhibit the interaction between platelets and fibrinogen are useful in modulating platelet thrombus formation.

In PCT/EP86/00012 (WO 86/04334), there is disclosed the use, for the manufacture of a medicament for regulating immune system responses of a peptide having the formula:

$$A-X-(B-Y)_n-C$$

in which X and Y are residues of amino acids or amino acid derivatives with positively charged side chains; A and C are substituents that preserve or augment the immuno-regulatory activity of the peptide; B is the residue of an amino acid or amino acid derivative that preserves or augments the immunoregulatory activity of the peptide; and n is O or l.

Moreover, in EP 0319506 A2, there is described a tetrapeptide derivative having inhibitory activity toward platelet aggregation, selected from the group consisting of X-Gly-Asp-Y in which X is a group of formula

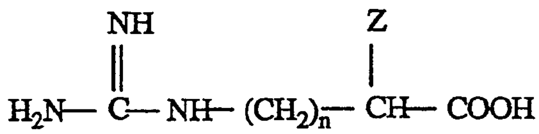

or Ac-Arg; Z is H, $NH_2$ or NH-acyl; n is 1 to 4; and Y is $H_2N-C(R_1)(R_2)(CH_2)_m R_3$, Tyr-$NH_2$ or Phe-$NH_2$ in which $R_1$ is H, alkyl, phenyl or phenylalkyl, $R_2$ is H, COOH, $CONH_2$, $COOCH_3$, $CH_2OH$, $CH_2NH_2$, $C(NH)CH_3$ or $C(NH)NH_2$ and $R_3$ is phenyl, biphenyl or naphthyl, each substituted with 1 to 3 alkyl or alkoxy groups, or an unsubstituted naphthyl or pyridyl group; and m is 0 to 2; in which alkyl and alkoxy each have 1 to 4 carbons; provided that, when Y is Tyr-$NH_2$ or Phe-$NH_2$, X is Ac-Arg; and further provided that, when Z is $NH_2$ or NH-acyl, X is in the D- or L- amino acid stereo-configuration.

We have now discovered certain new compounds which provide outstandingly good inhibition of platelet aggregation.

Accordingly, the present invention provides a compound having the formula I:

$$X-(Y)_x-Asp-Z \qquad (I)$$

in which X is a group of formula:

$$R_1-(CH_2)_m-R_2-(CH_2)_n-CH(R_3)-CO-$$

in which $R_1$ is $NHR_4$ in which $R_4$ is H, acyl, alkoxycarbonyl or alkyl, $R_2$ is arylene, heteroarylene, alkenylene, $CH_2$, O or NH, or any combination of these, $R_3$ is H, $NH_2$ or NH-acyl, in the R- or S- configuration, and m and n are each O or an integer from 1 to 5. Y is a group of formula:

$$-NH-(CH_2)_y-CO$$

in which y is an integer ranging from 1 to 12; x is O or 1; Z is $NH_2$ or the radical of any of the common alpha-amino acids, preferably in their L-configuration, or the amides of such amino acids; and pharmacologically - compatible salts of the compound of formula (I); provided that, when Y is Gly, X is other than Lys.

In the compound of formula I, Asp has the formula:

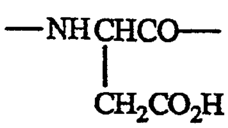

and is preferably of the L-configuration.

The group Z, when it denotes an alpha amino acid, is the radical of one of the following amino acids, which are in the L-enantiomeric form, unless otherwise stated:

| Amino Acid | Abbreviation |
|---|---|
| glycine | Gly |
| L-alanine | Ala |
| L-valine | Val |
| L-leucine | Leu |
| L-isoleucine | Ile |
| L-proline | Pro |
| L-methionine | Met |
| L-cysteine | Cys |
| L-phenylalanine | Phe |
| L-tyrosine | Tyr |
| L-tryptophan | Trp |
| L-histidine | His |
| L-lysine | Lys |
| L-arginine | Arg |
| L-aspartic acid | Asp |
| L-aspargine | Asn |
| L-glutamic acid | Glu |
| L-glutamine | Gln |
| L-serine | Ser |
| L-threonine | Thr |
| Gamma aminobutyric acid | GABA |

The amino acid radicals Z may be in the acid (OH) form or the amide ($NH_2$) form.

As can be seen, the group X in the compound of formula (I) is not a common alpha-amino acid. Rather, the group X is the radical of an alpha-amino acid which has been modified by extending its chain length by incorporating a group $R_2$ into the side chain of a common amino acid; by modifying or even removing the $NH_2$ group at $R_3$; and/or by modifying the terminal $NH_2$ to form a group $NHR_4$.

When $R_1$ is a group $NHR_4$, the substituent $R_4$ may be H, or any conventional amino-group protecting group. Examples of such protecting groups are, e.g. acyl, e.g. $C_1$-$C_{12}$ alkanoyl such as formyl, acetyl, n-propionyl, n-butyryl, n-pentanoyl, n-hexanoyl, n-octanoyl, n-decanoyl or n-dodecanoyl, or $C_6$ or $C_{10}$ aroyl - e.g. benzoyl or naphthoyl each optionally substituted by e.g. halogen atoms; $C_1$-$C_7$- alkoxy carbonyl e.g. $C_1$-$C_6$ alkoxy carbonyl such as methoxycarbonyl, ethoxycarbonyl, propoxycarbonyl, t-butoxycarbonyl or n-hexoxycarbonyl; $C_7$-$C_{11}$ -aralkyloxycarbonyl e.g. benzyloxycarbonyl; or $C_1$ -$C_4$ alkyl e.g. methyl, ethyl, n-propyl, isopropyl or n-butyl.

When $R_2$ is arylene, it is preferably $C_6$- or $C_{10}$ arylene, e.g. naphthylene or, preferably, o-, m- or p-phenylene.

Heteroarylene residues $R_2$ are, e.g. pyridinylene, pyrazinylene, pyrimidinylene, pyridazinylene or quinolinylene.

Alkenylene residues $R_2$ are preferably $C_2$-$C_4$ alkenylene groups especially vinylene (-CH=CH-) or -CH=CH-CH=CH- groups.

When $R_3$ is NH-acyl, such groups are preferably NH-$C_1$-$C_6$ alkanoyl or NH-$C_6$- or NH-$C_{10}$- aroyl groups, e.g. NH-formyl, NH-acetyl, NH-propionyl, NH-butyroyl, NH-benzoyl or NH-naphthoyl groups.

In the group NH-$(CH_2)_y$-CO in which y is an integer from 1 to 12, y is preferably an integer from 1 to 7.

One preferred sub-group of compounds of formula I is that in which X is $H_2N(CH_2)_5$ $CH(NH_2)$ CO or $H_2N(CH_2)_6$ CO, y is NH-$CH_2$-CO, Y is 1 and Z has its previous significance, namely compounds having the formula IA:

$$X^1\text{—Gly—Asp—}Z \qquad \text{(IA)}$$

in which $X^1$ is a group having the formula $H_2N(CH_2)_5$ $CH(NH_2)$ CO, viz h-Lys, or the formula $H_2N$ $(CH_2)_6$ CO, and Z has its previous significance, but is preferably Phe-OH or Trp-OH.

Of the compounds of formula IA, the most preferred compound is 7-amino-heptanoyl-Gly-L-Asp-L-Phe-OH having the formula:

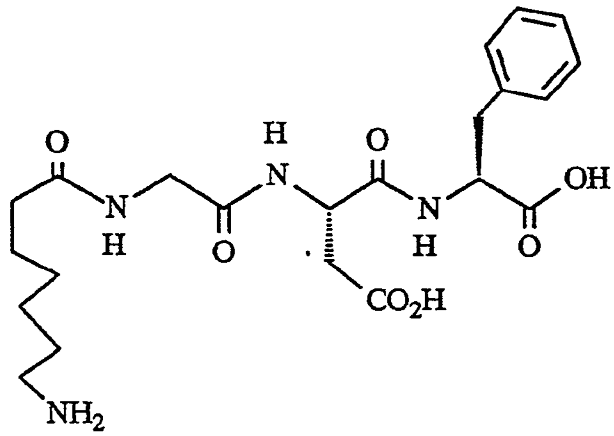

(IA[1])

Another preferred sub-group of compounds of formula I is that in which X is $H_2N\ (CH_2)_3\ CH_2CO$, $H_2N-(CH_2)_4CH_2CO$ or $H_2N-(CH_2)_5CH_2CO$, y is 3, and Z has its previous significance, namely compounds having the formula IB:

$$H_2N\text{-}(CH_2)_{m^1}—CH_2—(CH_2)_{n^1}—CH_2O—NH(CH_2)_3CO—Asp—Z \quad (IB)$$

in which $m^1$ and $n^1$ are the same or different and each is 0, 1, 2, 3 or 4, provided that $m^1 + n^1 = 2$, 3 or 4, and Z has its previous significance and is preferably Phe-OH or Trp-OH.

A further preferred sub-group of compounds of formula I is that in which X is $H_2N\ (CH_2)_9$ CO or $H_2N\ (CH_2)_{10}$ CO, y is O and Z has its previous significance, namely compounds having the formula IC:

$$H_2N\text{-}(CH_2)_{m''}CH_2(CH_2)_{n''}CH_2\ CO\text{-}Asp\text{-}Z \quad (IC)$$

m which $m^{11}$ and $n^{11}$ are integers ranging from 1 to 5 such that $m^{11} + n^{11} = 8$ or 9, and Z has its previous significance but is preferably Phe-OH or Trp-OH.

The novel peptides of the invention can be made by conventional methods of peptide synthesis.

The preferred method uses a solid phase procedure, using the 4-(hydroxymethyl)phenoxymethyl-copoly(styrene-1 %divinylbenzene) resin originally described by Wang, J. Amer. Chem. Soc. 95 1328 (1973) and improved by Merrifield, J. Org. chem. 46, 3433 (1981). The peptide is formed by a stepwise coupling of amino acids starting from the C -terminal end. For example phenylalanine can be attached to the resin first.

α-Amino acids have fluorenylmethoxycarbonyl (FMOC) protection at the a-amino position during coupling of the adjacent amino acid. Other amino acids can be protected at the amino position by any convenient group e.g., fluorenylmethoxycarbonyl, benzyloxycarbonyl (Cbz), t-butoxycarbonyl (Boc) etc. Side chain protection can be achieved using any suitable group e.g. aspartic acid can be protected on the side chain carboxylic acid as the t-butyl ester.

When the desired peptide has been assembled, it may be cleaved from the resin by treatment with a reagent such as trifluoroacetic acid water (90: 10) or trifluoracetic acid dichloromethane (90: 10).

The liberated peptide may then be purified e.g. by high performance liquid chromatography, or by other suitable purification methods.

The compounds of formula I according to the present invention have anti-thrombotic activity, viz they inhibit platelet aggregation and thrombus formation in human blood. Consequently, the compounds of the invention have value in the treatment of diseases such as strokes and myocardial infarctions. This platelet-binding inhibitor activity has been demonstrated in a biological screening procedure based on the aggregation of chymotrypsin-treated platelets. In this screen, compounds of the invention showed an activity comparable or even superior to that of the most potent natural compound, namely Echistatin, known to date.

The compounds of formula I may be administered to a patient orally or parenterally. The administration of the active compounds of formula I to the patient may be effected by means of any conventional pharmaceutical preparation. The pharmaceutical preparation will contain one or more pharmacologically - compatible carriers for the active material, which are inert to the active material.

The pharmaceutical preparations according to the invention, which contain compounds of the formula I or pharmaceutically acceptable salts thereof, may be those for enteral, such as oral or rectal, and parenteral, administration to humans that contain the pharmacologically active substance alone or together with a pharmacologically acceptable carrier. The dosage of the active substance depends on the age and the individual condition, and on the method of administration. In general, a compound of, formula I may be administered in dosages of approximately 1 to 200 mg per dosage unit or higher, with daily doses of about 0.02-5 mg/kg of

body weight.

The new pharmaceutical preparations contain, for example, from approximately 10 % to approximately 80 % preferably from approximately 20 % to approximately 60 % of the active substance. Pharmaceutical preparations according to the invention for enteral or parenteral administration are, for example, those in dosage unit forms, such as dragees, tablets, capsules, suppositories or ampoules. These are produced in a manner known per se, for example by means of conventional mixing, granulating, coating, dissolving or lyophilising processes. Thus, pharmaceutical preparations for or administration can be obtained by combining the active substance with solid carriers, optionally granulating a resulting mixture, and processing the mixture or granulate to form tablets or dragee cores, if desired or necessary after the addition of suitable adjuncts.

Suitable carriers are, especially, fillers, such as sugars, for example lactose, saccharose, mannitol or sorbitol, cellulose preparations and/or calcium phosphate, for example tricalcium phosphate or calcium hydrogen phosphate, or binders, such as starch pastes using, for example, corn, wheat, rice or potato starch, gelatin, tragacanth, methylcellulose and/or polyvinylpyrrolidone, and/or if desired, disintegrators, such as the abovementioned starches, and also carboxymethylstarch, cross-linked polyvinylpyrrolidone, agar, alginic acid or a salt thereof, such as sodium alginate. Adjuncts are, especially, flow-regulating agents and lubricants, for example, silica, talc, stearic acid or salts thereof, such as magnesium or calcium stearate, and/or polyethylene glycol. Dragee cores are provided with suitable, optionally gastric juice-resistant coating, there being used, inter alia, concentrated sugar solutions, which optionally contain gum arabic, talc, polyvinylpyrrolidone, polyethylene glycol and/or titanium dioxide, lacquer solutions in suitable organic solvents or solvent mixtures or, to produce gastric juice-resistant coating, solutions of suitable cellulose preparations, such as acetylcellulose phthalate or hydroxypropylmethylcellulose phthalate. Colouring matter or pigments may be added to the tablets or dragee coating, for example for identification purposes or for indicating different doses of active substance.

Other pharmaceutical preparations for oral administration are dry-filled capsules made of gelatin, and soft sealed capsules consisting of gelatin and a plasticiser, such as glycerin or sorbitol. The dry-filled capsules may contain the active substance in the form of a granulate, for example, in admixture with fillers, such as lactose, binders, such as starches, and/or glidants, such as talc or magnesium stearate, and optionally, stabilisers. In soft capsules, the active substance is preferably dissolved or suspended in suitable liquids, such as fatty oils, paraffin oil or liquid polyethylene glycols, or stabilisers may be added.

There come into consideration, as rectally administerable pharmaceutical preparations, for example, suppositories consisting of a combination of the active substance with a suppository base. Suitable suppository bases are, for example, natural or synthetic triglycerides, paraffin hydrocarbons, polyethylene glycols or higher alkanols. It is also possible to use gelatin rectal capsules that contain combination of the active substance with base substances, for example, liquid triglycerides, polyethylene glycols or paraffin hydrocarbons.

Especially suitable forms for parenteral administration are aqueous solutions of an active substance in water-soluble form, for example, a water-soluble salt, or suspensions of the active substance, such as corresponding oily injection suspensions, suitable lipophilic solvents or vehicles, such as fatty oils, for example sesame oil, or synthetic fatty acid esters, for example ethyl oleate or triglycerides, or aqueuos injection suspensions that contain viscosity-increasing substances, for example, sodium carboxymethylcellulose, sorbitol and/or dextran and optionally also stabilisers.

The invention is illustrated by the following Examples.

Example 1:7-Aminoheptanoyl-Gly-Asp-Phe-OH

10 grams of 4-(hydroxymethyl)phenoxy methyl resin (5.9 mmol of amino groups) with phenylalanine already attached and deprotected, is treated with a mixture, consisting of 3 equivalents each of Fmoc - Asp(OtBu) OH, Benzotriazole- 1-yl-oxy-tris-pyrrolidino-phosphonium-hexafluorophosphate (pyBop) and disopropylethylamine. Agitation is achieved by bubbling nitrogen through the mixture and reaction is carried out for 30 minutes. The resin is filtered and washed repeatedly with dimethylacetamide (DMA). The FMOC group is removed by treatment with piperidine in DMA for 10 minutes, the resin filtered and washed repeatedly with DMA before coupling the next amino acid derivative. The cycle described above is followed for attachment of Gly and 7-aminoheptanoic acid with the exception that the reaction time is increased to 60 mins for FMOC aminoheptanoic acid.

With the assemblage of the peptide chain complete, the product is cleaved from the resin. This is achieved by repeated treatments with 90 % TFA in dichloromethane, resulting in concomitant deprotection of the Aspartic t-butyl group. The peptide is purified by reverse phase HPLC using a Zorbax-C8 preparative column and eluting with 15 % acetonitrile in water (0.1 % TFA) to give 1.3 g of pure compound.

AAA : Gly(1.00), Asp(0.97), Phe(1.01)

Examples 2 to 5 were prepared by the procedure described in Example 1 but using the appropriate amino

acid derivatives.

Example 2: 2,7-Diaminoheptanoyl-Gly Asp-Phe-OH

AAA: homoLys(1.00), Gly(1.00), Asp(1.05), Phe(0.93)

Example 3: 10-Aminodecanoyl-Asp-Phe-OH

FAB-MS: (M+H)+=450

Example 4: 11-Aminoundecanoyl-Asp-Phe-OH

FAB-MS: (M+H)+=464

Example 5: 6-Aminohexanoyl-GABA-Asp-Phe-OH

FAB-MS: (M+H)+=479

The platelet-binding inhibitor activity of the compounds of this invention can be demonstrated by various assays. In one assay the ability of the compounds to inhibit the aggregation of chymotrypsin treated platelets is measured.

In another test their ability to inhibit the attachment of activated platelets to solid phase fibrinogen is measured using a sandwich ELISA.

In both tests some of the compounds demonstrate considerably more activity than the tetrapeptides Arg-Gly-Asp-Ser and Arg-Gly-Asp-Val(see tables 1 and 2) and in one test (table 1) equipotency with the snake venom polypeptide echistatin.

For both methods of test blood platelets are isolated from citrated whole blood and plasma by differential centrifugation, treated with the protease chymotrypsin, fixed with paraformaldehyde and stored at -20°C.

Example 6: Aggregation

The quantitated aggregation response is initiated by the addition of both Calcium and fibrinogen and the response recorded using an optical aggregometer. The extent of inhibition by agents interfering with the fibrinogen binding to platelet receptors is determined at fixed concentrations of such reagents. The effect on the extent of the control aggregatory response is quantified at a given time after the addition of the fibrinogen. The results are as follows.

Table 1

| Compound | IC50(uM) |
|---|---|
| Echistatin | 0.025 |
| Arg-Gly-Asp-Ser | 20 |
| Arg-Gly-Asp-Val | <10 |
| Example 1 | 0.01-0.1 |
| Example 2 | 0.1-1 |

Example 8: Sandwich ELISA assay

The binding of chymotrypsin-treated fixed platelets to solid phase fibrinogen is measured in microlitre plates

using a sandwich ELISA. The sandwich consists of a mouse anti-human CD9 monoclonal antibody which binds to the platelets, followed by a rabbit anti-mouse immunoglobulin G conjugated to the enzyme alkaline phosphatase. The phosphatase substrate is p-nitrophenyl phosphate whose cleavage product is detected by absorbance at 405 nm. The effect of agents that interefere with the binding of fibrinogen to platelets can be determined at fixed concentrations of such agents. For further details of the method refer to Nieman, C.J et al, Thrombosis Res. submitted for publication. The results obtained are as follows.

Table 2

| Compound | IC50(uM) |
|---|---|
| Echistatin | 0.1 |
| Arg-Gly-Asp-Ser | 135 |
| Arg-Gly-Asp-Val | 25 |
| Example 1 | 2 |
| Example 3 | 1 |
| Example 4 | 5 |
| Example 5 | 50 |

## Claims

1. A compound having the formula I:

$$X—(Y)_x—Asp—Z \qquad (I)$$

in which X is a group of formula:

$$R_1—(CH_2)_m—R_2—(CH_2)_n—CH(R_3)—CO—$$

in which $R_1$ is $NHR_4$ in which $R_4$ is H, acyl, alkoxycarbonyl or acyl, $R_2$ is arylene, heteroarylene, alkenylene, $CH_2$, O or NH, or any conbination of these, $R_3$ is H, $NH_2$ or NH-acyl, in the R- or S- configuration, and m and n are each O or an integer from 1 to 5; Y is a group of formula:

$$NH—(CH_2)_y—CO$$

in which y is an integer ranging from 1 to 12; Z is $NH_2$ or the radical of any of the common alpha-amino acids, or of the amides of such amino acids; and pharmacologically - compatible salts of the compound of formula I; provided that, when Y is Gly, X is other than Lys.

2. A compound according to claim 1 in which when Z is the radical of an alpha-amino acid, such acid is in the L-configuration.

3. A compound according to claim 1 or 2 in which the group Asp is in the L-configuration.

4. A compound according to any of the preceding claims in which y is an integer ranging from 1 to 7.

5. A compound according to claim 1 having the formula IA:

$$X^1 - Gly - Asp - Z \qquad (IA)$$

in which $X^1$ is a group having the formula:

$H_2N(CH_2)_5CH(NH_2)\ CO$ (viz h-Lys)

or the formula:

$H_2N(CH_2)_6\ CO$

and Z is defined in claim 1.

6. A compound of formula IA according to claim 5 in which Z is Phe-OH or Trp-OH.

7. A compound of formula IA according to claim 5 which is 7-amino-heptanoyl-Gly-L-Asp-L-Phe-OH.

8. A compound of formula IA according to claim 5 which is h-Lys-Gly-Asp-Phe-OH.

9. A compound according to claim 1 having the formula IB:

$$H_2N\text{-}(CH_2)_{m^1} - CH_2 - (CH_2)_{n^1} - CH_2O - NH(CH_2)_3CO - Asp - Z \qquad (IB)$$

in which $m^1$ and $n^1$ are the same or different and each is 0,1,2,3 or 4, provided that $m^1 + n^1 = 2,3$, or 4, and Z is as defined in claim 1.

10. A compound of formula IB according to claim 9 in which Z is Phe-OH or Trp-OH.

11. A compound according to claim 1 having the formula IC:

$$H_2N\text{-}(CH_2)_{m''}CH_2(CH_2)_{n''}CH_2CO\text{-}Asp\text{-}Z \qquad (IC)$$

in which $m^{11}$ and $n^{11}$ are integers ranging from 1 to 5, such that $m^{11} + n^{11} = 8$ or 9 and Z is as defined in claim 1.

12. A compound of formula IC according to claim 1 in which Z is Phe-OH or Trp-OH.

13. A pharmaceutical composition containing a compound of formula I of claim 1 and customary carriers.

14. A compound of the formula I of claim 1 for use in a method for the therapeutic treatment of the human or animal body.

15. A compound of formula I according to claim 1 for use in the inhibition of platelet aggregation.

16. The use of compounds of formula I according to claim 1 and customary carriers for producing pharmaceutical preparations.

17. Process for the production of a compound and pharmcologically-compatible salts thereof, provided that when Y is Gly, X is other than Lys having the formula I as defined in claim 1, comprising the stepwise coupling of the relevant amino acid components of the compound of formula I or to the 4-(hydroxymethyl)phenoxymethyl-copoly(styrene-1 % divinylbenzene) resin starting at the C-terminal end and when the peptide compound of formula I has been assembled, it is cleaved from the resin by treatment with trifluoroacetic acid/water (90: 10) or trifluoroacetic acid/dichloromethane (90: 10).

18. Process according to claim 17, in which phenylalanine is attached to the resin first.

19. Process according to process claims 17 and 18 in which α-amino acids have fluorenylmethoxycarbonyl protection at the α-amino position, during coupling of the adjacent amino acid, and after amino acids are optionally protected at the amino portion by convenient protecting groups, e.g. fluorenylmethoxycarbonyl, benzyloxy carbonyl or t-butoxycarbonyl groups.

**Claims for the following Contracting States: ES and GR**

1. Process for the production of a compound having the formula I

$$X—(Y)_x—Asp—Z \quad (I)$$

in which X is a group of formula:

$$R_1—(CH_2)_m—R_2—(CH_2)_n—CH(R_3)—CO—$$

in which $R_1$ is $NHR_4$ in which $R_4$ is H, acyl, alkoxycarbonyl or acyl, $R_2$ is arylene, heteroarylene, alkenylene, $CH_2$, O or NH, or any conbination of these, $R_3$ is H, $NH_2$ or NH-acyl, in the R- or S- configuration, and m and n are each O or an integer from 1 to 5; Y is a group of formula:

$$NH—(CH_2)_y—CO$$

in which y is an integer ranging from 1 to 12; Z is $NH_2$ or the radical of any of the common alpha-amino acids, or of the amides of such amino acids; and pharmacologically - compatible salts of the compound of formula I; provided that, when Y is Gly, X is other than Lys, comprising the stepwise coupling of the relevant amino acid components of the compound of formula I or to the 4-(hydroxymethyl)phenoxymethyl-copoly(styrene- 1 % divinyl benzene) resin starting at the C-terminal end and when the peptide compound of formula I has been assembled, it is cleaved from the resin by treatment with trifluoroacetic acid/water (90: 10) or trifluoroacetic acid/dichloromethane (90: 10).

2. Process according to claim 1 wherein there are produced compounds of formula I in which when Z is the radical of an α-amino acid, such acid is in the L-configuration.

3. Process according to claim 1 wherein there are produced compounds of formula I in which the group Asp is in the L-configuration.

4. A process according to claim 1 wherein there are produced compounds of formula I in which Y is an integer ranging from 1 to 7.

5. A process according to claim 1 wherein there are produced compounds of formula IA

$$X^1—Gly—Asp—Z \quad (IA)$$

in which $X^1$ is a group having the formula:

$H_2N(CH_2)_5CH(NH_2)$ CO (viz h-Lys)

or the formula:

$H_2N(CH_2)_6$ CO

and Z is defined in claim 1.

6. A process according to claim 1 wherein there are produced compounds of formula IA as defined in claim 5 in which Z is Phe-OH or Trp-OH.

7. A process according to claim 1 wherein there is produced 7-amino-heptanoyl-Gly-L-Asp-L-Phe-OH as compound of formula IA.

8. A process according to claim 1 wherein there is produced h-Lys-Gly-Asp-Phe-OH as compound of formula IA.

9. A process according to claim 1 wherein there are produced compounds of formula IB

$$H_2N\text{-}(CH_2)_{m^1}—CH_2—(CH_2)_{n^1}—CH_2O—NH(CH_2)_3CO—Asp—Z \quad (IB)$$

in which $m^1$ and $n^1$ are the same or different and each is 0,1,2,3 or 4, provided that $m^1 + n^1 = 2,3$, or 4, and Z is as defined in claim 1.

**10.** A process according for claim 1 wherein there are produced compounds of formula IB as defined in claim 9 in which Z is Phe-OH or Trp-OH.

**11.** A process according to claim 1 wherein there are produced compounds of formula IC

$$H_2N-(CH_2)_{m''}-CH_2(CH_2)_{n''}\ CH_2CO\text{-Asp-Z} \qquad \text{(IC)}$$

in which $m^{11}$ and $n^{11}$ are integers ranging from 1 to 5, such that $m^{11} + n^{11} = 8$ or 9 and Z is as defined in claim 1.

**12.** A process according to claim 1 where there are produced compounds of formula IC as defined in claim 11 in which Z is Phe-OH or Trp-OH.

**13.** A process according to claim 1, in which phenylalanine is attached to the resin first.

**14.** A process according to claim 1 to 13 in which α-amino acids have fluorenylmethoxycarbonyl protection at the α-amino position, during coupling of the adjacent amino acid, and other amino acids are optinally protected at the amino position by fluorenylmethoxycarbonyl, benzyloxycarbonyl or t-butoxycarbonyl groups.